# EUROPEAN PATENT APPLICATION

(11) **EP 2 568 046 A1**
(43) Date of publication of application: **13.03.2013**
(21) Application number: 11306102.2
(22) Date of filing: 06.09.2011
(51) Int. Cl.: C12N 15/113, A61P 31/22

(54) **MicroRNAs from human herpesvirus 6**

(71) Applicant: Centre National de la Recherche Scientifique (C.N.R.S), 75016 Paris (FR)
(72) Inventor: Tuddenham, Lee, 67000 Strasbourg (FR); Pfeffer, Sébastien, 67000 Strasbourg (FR); Dolken, Lars, 81475 München (DE); Jung, Jette, 80539 München (DE)
(74) Representative: Blot, Philippe Robert Emile

(57) **Abstract**

The present invention concerns microRNAs and other small non-coding RNAs from human herpesvirus 6 (HHV-6B). Use of said RNAs as therapeutic targets and diagnostic agents is provided, as are antagonists of said RNAs and their therapeutic uses.

## Description

### FIELD OF THE INVENTION

The present invention concerns microRNAs and other small non-coding RNAs from human herpesvirus 6 (HHV-6B). Use of said RNAs as therapeutic targets and diagnostic agents is provided, as are antagonists of said RNAs and their therapeutic uses.

### BACKGROUND

Human herpesvirus 6 (HHV-6) is one of eight herpesviruses known to infect humans, and was first isolated in 1986 from immunocompromised patients suffering from lymphoproliferative disorders. HHV-6 is a ubiquitous human pathogen with seroprevalence rates exceeding 95% in industrialized nations. Together with HHV-7, it belongs to the *Roseolovirus* genus within the betaherpesvirus subfamily. It exists as two variants: HHV-6A and HHV-6B, which differ in respect to their biological properties, tropisms, and clinical manifestations. In 1988, HHV-6B was identified as the causative agent of the childhood disease, exanthem subitum (roseola or 3-day fever). Primary infection typically presents as an acute febrile illness, sometimes followed by a red rash. Additional symptoms may include otitis, meningitis and seizures. While severe complications resulting from primary infection such as encephalitis and encephalopathy are rare, reactivation of HHV-6 in transplant patients (particularly those receiving hematopoietic stem cells) can present with life-threatening complications, most notably due to encephalitis. In addition, HHV-6 may affect the progression of other viral infections, trans-activating both HHV-7, HCMV, and HIV-1, which generally results in poorer prognosis. Finally, HHV-6 has been implicated in a range of neurological diseases, most prominently multiple sclerosis (MS), although a causative role of HHV-6 in MS pathology remains controversial.

The HHV-6 genome consists of a linear, double-stranded DNA molecule, 160 to 162 kb long, comprising a unique region (U) of 145-kb flanked by terminal direct repeats (DR_{L} and DR_{R}) of 8 to 9 kb. Unlike all other human herpesviruses, HHV-6A has been categorically proven to integrate into host telomeres as a method of latency in individuals with chromosomally integrated HHV-6 (CIHHV-6), rather than residing as an episome. Importantly the virus can be reactivated from this state, and be passed on through generations. HHV-6 has a broad cellular tropism, as its receptor, CD46 is expressed on all nucleated cells. The sites of latency are not precisely defined yet, but may consist of CD4⁺ T cells, monocytes and early bone marrow progenitor cells. Nonetheless, it has been shown to preferentially infect CD4 T cells which also represent a major site of latency.

HHV-6, like HCMV (although less well studied) uses a multitude of approaches to modulate the host-pathogen environment and establish latency. Previous studies have focused on the role of viral proteins that modulate MHC-class I gene expression, and viral chemokines and receptors that interact with the host. Recently, many members of the herpesvirus family have been shown to express their own microRNAs (microRNAs). Since the seminal discovery of microRNAs in Epstein-Barr virus, more than 235 microRNAs of viral origin have been identified and listed in the microRNA repository: miRBase (http://www.mirbase.org/). Conserved throughout metazoan evolution, microRNAs derive from large primary transcripts (pri-microRNA) that form local hairpin structures. The pri-microRNA is sequentially processed by the nuclear and cytoplasmic RNase III enzymes Drosha and Dicer to generate the mature microRNA of around 22 nucleotides. This microRNA becomes functional only after its incorporation into an Argonaute (Ago) containing microRNP, also known as RISC (RNA induced silencing complex), where it will mediate the regulation of target messenger mRNAs via binding to partially complementary sites typically located in their 3'-UTRs.

The vast majority of virus-encoded microRNAs derive from herpesviruses, and so far HHV-3, HHV-6 and HHV-7 are the only herpesviruses for which microRNAs have not been characterized. For HHV-3, deep-sequencing of latently infected trigeminal ganglia failed to identify any HHV-3 microRNAs; this data was in support of computational data which predicted that HHV-6, HHV-7 and HHV-3 are unlikely to encode for microRNAs based on secondary structure predictions (Pfeffer et al, 2005, Nat Methods 2(4) 269-276). To resolve the question as to whether HHV-6 encodes for microRNAs, we performed small RNA cloning followed by massive parallel sequencing to identify the small RNAs deriving from the virus during lytic infection.

### DESCRIPTION OF THE INVENTION

The inventors have cloned small RNAs both from total RNA isolated from CD4⁺ T lymphocyte cell line (Sup-T-1) infected with a laboratory strain of HHV-6B, and from RNAs co-immunoprecipitated with Ago2. They have identified three microRNAs from HHV-6B, all of which are conserved in the closely related HHV-6A, but display intervariant as well as interstrain sequence variation. These viral microRNAs are encoded at two positions within the genome (in both DR_{L} and DR_{R}). One of them is a seed orthologue of miR-582-5p, a human microRNA not expressed in Sup-T-1 T-cells. In addition, the inventors have identified a novel viral transcript deriving from the minimal efficient origin of lytic replication (OriLyt) of 60-65 nucleotides that gives rise to abundant RNA species of 18 and 19 nucleotides in length that may represent a new class of origin-derived viral small RNAs.

Thus, in a first aspect, the invention provides a nucleic acid molecule comprising the sequence of an HHV-6 microRNA or the complement thereof. Preferably, the molecule is an isolated nucleic acid molecule. In preferred embodiments, said microRNA has the sequence of a nucleic acid sequence shown in Tables 2 to 6 or Figs 10 and 11 (SED ID No 1-28), or the complement thereof.

In some embodiments, the nucleic acid molecule is RNA. In other embodiments, the nucleic acid molecule is DNA.

In some embodiments, said HHV-6 microRNA is a precursor microRNA. In one embodiment, the HHV-6 microRNA is part of a hairpin precursor sequence of fragment thereof. In other embodiments, said HHV-6 microRNA is a mature microRNA.

In some embodiments said HHV-6 microRNA is a HHV-6B-miR-H-A microRNA (SEQ ID No 1 or 10), a HHV-6B-miR-H-B microRNA (SEQ ID No 3, 11, 12 or 13), a HHV-6B-miR-H-C-3p microRNA (SEQ ID No 6, 14, 15, 16, 17, 18, or 19), or a HHV-6B-miR-H-C-5p microRNA (SEQ ID No 5, 20, 21 or 22).

In certain embodiments, the invention provides an isolated nucleic acid molecule comprising a nucleic acid sequence shown in Tables 2 to 6 or Figs 10 and 11, (SED ID No 1-28), or the complement thereof. In some embodiments, said nucleic acid molecule consists of a nucleic acid sequence shown in Tables 2 to 6 or Figs 10 and 11, (SED ID No 1-28), or the complement thereof.

Also provided are variants and fragments of an HHV-6 microRNA, in particular naturally occurring variants and fragments.

Also provided are antagonists of an HHV-6 microRNA. In some embodiments, said antagonist is an HHV-6 microRNA antisense nucleic acid such as an antagomir, preferably a Locked Nucleic Acid (LNA) oligonucleotide. In preferred embodiments, an HHV-6 microRNA antisense nucleic acid comprises or consists of a sequence complementary to a nucleic acid sequence shown in Tables 2 to 6 (SED ID No 1-28), or a fragment thereof.

Also provided is a method of identifying a candidate HHV-6 microRNA antagonist, said method comprising contacting a test compound with an HHV-6 microRNA and determining the presence or absence of a complex comprising said test compound and said microRNA, wherein the presence of said complex is indicative of the suitability of said test compound as an HHV-6 antagonist. Preferably, said method is an *in vitro* method.

Also provided are vectors comprising the nucleic acids of the invention. Typically, the vector will comprise the hairpin precursor sequence containing the mature HHV-6 microRNA sequence. The vector may be, for example, a plasmid, a cosmid, a phage or a viral vector, such as a DNA or RNA virus. A DNA virus can be a single stranded or double stranded DNA virus. The vector may further include a selectable marker, such as for instance a drug resistance marker or a detectable gene marker, such as β-galactosidase.

Also provided are methods for inhibiting microRNP activity in a cell, said methods comprising contacting said cell with an HHV-6 antagonist as described herein. In one aspect, the invention relates to a method for inhibiting the activity of a microRNP comprising an HHV-6 microRNA, comprises introducing contacting said microRNP or cell containing said microRNP with an HHV-6 antisense microRNA molecule as disclosed above, which antisense RNA is complementary to said microRNA molecule. MicroRNP activity refers to the cleavage or the repression of translation of the target sequence. The target sequence may be any sequence which is partially or perfectly complementary to the sequence of bases in the HHV-6microRNA microRNA. The target sequence can be, for example, a viral or host messenger RNA.

In some embodiments, said methods are *in vitro* or *ex vivo* methods. Alternatively, said methods may be *in vivo* methods, for example methods of treatment of an HHV-6 infection or HHV-6-related disease.

Also provided are methods of down-regulating expression an HHV-6 or cellular protein in a cell using the nucleic acids and antagonists disclosed herein. In one embodiment, said HHV-6 protein expression is expression within a cell, such as a mammalian cell, and said method comprises introducing said nucleic acid or antagonist into said cell. In a preferred embodiment, said HHV-6 protein is HHV-6 B1 or B2 or B3 protein. In a preferred embodiment, said nucleic acids include HHV-6B-miR-H-A microRNA (SEQ ID No 1 or 10), HHV-6B-miR-H-B microRNA (SEQ ID No 3, 11, 12 or 13), HHV-6B-miR-H-C-3p microRNA (SEQ ID No 6, 14, 15, 16, 17, 18, or 19), or HHV-6B-miR-H-C-5p microRNA (SEQ ID No 5, 20, 21 or 22), as disclosed herein.

Further provided are methods of diagnosis comprising detection of an HHV-6 microRNA. Said methods may comprise use of the nucleic acids described herein. In one embodiment, said methods comprise detection of HHV-6 microRNA in a biological sample from a patient. In preferred embodiments, said methods comprise detection of an HHV-6B specific microRNA in a biological sample from a patient.

Also provided are methods of diagnosis using the nucleic acids described herein.

In some embodiments, said methods are methods of diagnosis of an HHV-6 infection, including a latent HHV-6 infection. In some embodiments, said methods are methods of detecting an HHV-6A or an HHV-6B infection and/or of classifying an HHV-6 infection as HHV-6A or an HHV-6B infection. In some embodiments, said methods may be methods of diagnosing an individual at risk of developing an HHV-6-related condition. In further embodiments, said methods of diagnosis are methods of diagnosis of tumour, in particular pituitary adenoma.

In some embodiments, said method of diagnosis is a method of classifying a tumour comprising detection of a nucleic acid of the invention in a tumour or biological sample.

Preferably, the diagnostic methods of the invention are *in vitro* or *ex vivo* methods. Alternatively, said methods may be *in vivo* methods.

Also provided are methods of prognosis of patients suffering from a non-HHV-6 viral infection, in particular a HHV-7, HCMV, or HIV-1 infection, which methods comprise detection of an HHV-6 microRNA in a biological sample from said patient, wherein detection of said HHV-6 microRNA is associated with a poorer prognosis of said infection.

Also provided are therapeutic methods using or targeting the nucleic acids of the invention. In one aspect, the invention provides a method of treating an HHV-6 infection comprising administering to a subject an HHV-6 microRNA antagonist as disclosed herein. Preferably, the HHV-6 microRNA antagonist is administered in a therapeutically effective amount and the subject is a patient in need thereof.

Also provided is an HHV-6 microRNA or other nucleic acid disclosed herein for use in a method of medical treatment.

Treatment includes treatment of and/or an HHV-6 infection, including a latent infection, a tumour or other condition related to an HHV-6 infection, as described below. In some embodiments, said treatment is treatment of an immunocompromised patient, such as a transplant recipient taking immunosuppressive drugs. For example, said treatment may be the prevention of graft rejection in a transplant recipient.

In some embodiments, said treatment may be targeted specifically at HHV-6A or HHV-6B.

Also provided is a method of overexpression of an HHV-6 microRNA comprising introducing a vector comprising a nucleic acid as described herein into a cell, preferably under conditions suitable for overexpression of said microRNA. The vector is preferably a DNA plasmid or viral vector.

Also provided is a method of detection of HHV-6 infection of a subject comprising detection of an HHV-6 microRNA in a biological sample from said subject. Preferably, the subject is a human subject.

The subject is preferably a human subject.

A biological sample may be, for example, a blood, serum, plasma, cerebrospinal fluid, urine, semen, saliva, biopsy or other tissue sample

The molecules of the present invention can be introduced into a cell, or used *in vitro,* to study the function of HHV-6 microRNA. Any DNA viral microRNA molecule and/or anti-DNA viral microRNA molecule mentioned above can be introduced into a cell for studying their function. In one embodiment, a microRNA in a cell is inhibited with a suitable anti-microRNA molecule. Alternatively, the activity of a microRNA molecule in a cell can be enhanced by introducing into the cell one or more additional microRNA molecules. The function of the microRNA can be inferred by observing changes in RNA or protein levels and phenotypic changes associated with inhibition and/or enhanced activity of the microRNA in the cell. The molecules of the present invention may also be used in the analysis of the regulation of miRNA molecule biogenesis.

Preferably, said methods are *in vitro* or *ex vivo* methods. Alternatively, said methods may be *in vivo* methods.

### Nucleic acids

The term 'nucleic acid' will generally refer to at least one molecule or strand of DNA, RNA or a derivative or mimic thereof, comprising at least one nucleobase, such as, for example, a naturally occurring purine or pyrimidine base found in DNA (e.g., adenine 'A,' guanine 'G,' thymine 'T,' and cytosine 'C') or RNA (e.g. A, G, uracil 'U,' and C). The term 'nucleic acid' encompasses the terms 'oligonucleotide' and 'polynucleotide.' The term 'oligonucleotide' refers to at least one molecule of between about 3 and about 100 nucleobases in length. The term 'polynucleotide' refers to at least one molecule of greater than about 100 nucleobases in length. These definitions generally refer to at least one single-stranded molecule, but in specific embodiments will also encompass at least one additional strand that is partially, substantially or fully complementary to the at least one single-stranded molecule. Thus, a nucleic acid may encompass at least one double-stranded molecule or at least one triple-stranded molecule that comprises one or more complementary strand(s) or 'complement(s)' of a particular sequence comprising a strand of the molecule.

The HHV-6 microRNA molecules encompassed by the invention may be DNA or RNA molecules. The sequences of nucleic acid molecules shown in the Figures, Tables and SEQ ID Nos are shown having uracil bases. Uracil bases occur in unmodified RNA molecules. The invention also includes unmodified DNA molecules. The sequence of bases of the unmodified DNA molecule is the same as the unmodified RNA molecules, except that in the unmodified DNA molecule, the uracil bases are replaced with thymine bases. The invention also encompasses the DNA equivalents of the RNA sequences disclosed herein.

Also included within the scope of the invention are nucleic acid sequences complementary to the RNA sequences disclosed herein and their DNA equivalents. Such nucleic acids comprise a sequence complementary to that of any of the nucleic acid sequences described herein or analogues, fragments or variants thereof.

The invention also encompasses antisense and other antagonist nucleic acids as described below. Thus, the nucleic acids of the invention may include not only the sequences described herein but also antisense nucleic acids thereto.

The nucleic acid(s) of the present invention, regardless of the length of the sequence itself, may be combined with other nucleic acid sequences, including but not limited to, promoters, enhancers, polyadenylation signals, restriction enzyme sites, multiple cloning sites, coding segments, and the like, to create one or more nucleic acid construct(s). The overall length may vary considerably between nucleic acid constructs. Thus, a nucleic acid segment of almost any length may be employed, with the total length preferably being limited by the ease of preparation or use in the intended recombinant nucleic acid protocol.

In another embodiment, the invention relates to analogues of the HHV-6 microRNAs disclosed herein. In this embodiment, the microRNA molecule comprises a minimum number of moieties, preferably a minimum of 13, more preferably a minimum of 15, even more preferably a minimum of 18, and most preferably a minimum of 21 moieties, and/or a maximum number of 50 moieties, preferably a maximum of 40, more preferably a maximum of 30, even more preferably a maximum of 25, and most preferably a maximum of 23 moieties. A suitable range of minimum and maximum numbers of moieties may be obtained by combining any of the above minima with any of the above maxima.

Each moiety comprises a base bonded to a backbone unit. In this specification, a base refers to any one of the nucleic acid bases present in DNA or RNA. The base can be a purine or pyrimidine.

Variants of the nucleic acid sequences disclosed herein are also provided. Such variants may include nucleic acids that have at least about 80% nucleic acid sequence identity with a nucleotide sequence disclosed herein. Preferably, a variant nucleic acid will have at least about 80%, 81%, 82%, 83%, 84%, 85%, 86%, 87%, 88%, 89%, 90%, 91%, 92%, 93%, 94%, 95%, 96%, 97%, 98%, 99% nucleotide sequence identity to a full-length nucleotide sequence or a fragment of a nucleotide sequence as disclosed herein. Nucleotide sequence identity is defined as the percentage of nucleic acid residues in the variant sequence that are identical with the nucleic acid residues in the reference sequence, after aligning the sequences and introducing gaps, if necessary, to achieve the maximum percent sequence identity, and not considering any conservative substitutions as part of the sequence identity. Sequence identity may be determined over the full length of the variant sequence, the full length of the reference sequence, or both. Methods for sequence alignment and determination of sequence identity are well known in the art, for example using publicly available computer software such as BioPerl, BLAST, BLAST-2, CS-BLAST, FASTA, ALIGN, ALIGN-2, LALIGN, Jaligner, matcher or Megalign (DNASTAR) software and alignment algorithms such as the Needleman-Wunsch and Smith-Waterman algorithms.

Fragments of the nucleic acids disclosed herein are also encompassed by the invention. Such fragments may be truncated at the 5' end or the 3' end, or may lack internal bases, for example, when compared with a full length sequence disclosed herein. Preferably, said fragments are at least about 2, 3, 4, 5, 6, 7, 8, 9, 10, 11, 12, 13, 14, 15, 16, 17, 18, 19, 20, 21, 22, 23 or 24 bases or base pairs in length. In certain embodiments, said fragments are not more than about 12, 13, 14, 15, 16, 17, 18, 19, 20, 21, 22, 23, 24, 25, 26, 27, 28, 29, 30, 31, 32, 33, 34, 35, 40, 45, 50, 55, 60, or 65 bases or base pairs in length. A suitable range of minimum and maximum numbers of bases or base pairs may be obtained by combining any of the above minima with any of the above maxima.

A nucleic acid molecule of the invention is preferably isolated, which means that it is essentially free of other nucleic acids. 'Essentially free' from other nucleic acids means that the nucleic acid molecule is at least about 90%, preferably at least about 95%, preferably at least about 98% and more preferably at least about 99% free of other nucleic acids.

Preferably, the molecule is essentially pure, which means that the molecule is free not only of other nucleic acids, but also of other materials used in the synthesis and isolation of the molecule. Materials used in synthesis include, for example, enzymes. Materials used in isolation include, for example, gels, such as SDS-PAGE. The molecule is at least about 90% free, preferably at least about 95% free, preferably at least about 98% free, and more preferably at least about 99% free of other nucleic acids and such other materials.

### Modified nucleic acids

Also included within the scope of the invention are nucleic acids comprising one or more modifications. Said nucleic acids may include HHV-6 microRNA molecules as disclosed herein and antagonists thereto, including antisense molecules.

Preferably, the nucleic acids of the invention comprise at least one moiety which confers increased nuclease resistance, such as a moiety which is not recognised by a nuclease. Such modified moieties are well known in the art, and were reviewed, for example, by Kurreck, Eur. J. Biochem. 270, 1628-1644 (2003). The nuclease to which resistance is conferred may be an exonuclease, an endonuclease, or both. The exonuclease can be a 3'- '5' exonuclease or a 5'→3* exonuclease. Examples of 3'→5' human exonuclease include PNPT1, Werner syndrome helicase, RRP40, RRP41, RRP42, RRP45, and RRP46. Examples of 5'-→3 ' exonuclease include XRN2 and FENI. Examples of endonucleases include Dicer, Drosha, RNase4, Ribonuclease P, Ribonuclease HI, DHP1, ERCC-1 and OGG1. Examples of nucleases which function as both an exonuclease and an endonuclease include APEI and EXOI.

A modified moiety can occur at any position in the nucleic acid molecule. For example, to protect a microRNA molecule against 3'-→5' exonucleases, the molecule can have at least one modified moiety at the 3' end of the molecule, and preferably at least two modified moieties at the 3' end. If it is desirable to protect the molecule against 5'→3' exonuclease, the DNA virus microRNA molecule can have at least one modified moiety, and preferably at least two modified moieties at the 5' end of the molecule. The DNA virus microRNA molecule can also have at least one, and preferably at least two modified moieties between the 5' and 3' end of the molecule to increase resistance of the molecule to endonucleases. Preferably, at least about 10%, more preferably at least about 25%, even more preferably at least about 50%, and further more preferably at least about 75%, and most preferably about 95% of the moieties are modified. In one embodiment, all of the moieties are nuclease resistant.

In one embodiment, the nucleic acid molecule comprises at least one modified deoxyribonucleotide moiety. Modified deoxyribonucleotide moieties are known in the art, and include, for example, a phosphorothioate deoxyribonucleotide moiety. An oligonucleotide molecule comprising a phosphorothioate deoxyribonucleotide moiety may be referred to as phosphorothioate (PS) DNA. See, for example, Eckstein, Antisense Nucleic Acids Drug Dev. 10, 117-121 (2000). Another example is an N'3-N'5 phosphoroamidate deoxyribonucleotide moiety. An oligonucleotide molecule comprising phosphoroamidate deoxyribonucleotide moieties may be referred to as phosphoroamidate (NP) DNA. See, for example, Gryaznov et al., J. Am. Chem. Soc. 116, 3143-3144 (1994).

In another embodiment, the molecule comprises at least one modified ribonucleotide moiety. Modified ribonucleotide moieties are known in the art, and include, for example, a ribonucleotide moiety that is substituted at the 2' position. The substituents at the 2' position may, for example, be a C₁ to C₄ alkyl group. The C₁ to C₄ alkyl group may be saturated or unsaturated, and unbranched or branched. Some examples of C₁ to C₄ alkyl groups include ethyl, isopropyl, and allyl. The preferred C₁ to C₄ alkyl group is methyl. An oligoribonucleotide molecule comprising ribonucleotide moieties substituted at the 2' position with a C alkyl group is generally referred to as a 2'-O -(C₁-C₄ alkyl) RNA, e.g., 2'-O-methyl RNA (OMe RNA). Another example of a substituent at the 2' position of a modified ribonucleotide moiety is a C₁ to C₄ alkoxy - C₁ to C₄ alkyl group. The C₁ to C₄ alkoxy (alkyloxy) and C₁ to C alkyl group may comprise any of the alkyl groups described above. The preferred C₁ to C₄ alkoxy - to C alkyl group is methoxyethyl. An oligonucleotide molecule comprising more than one ribonucleotide moiety that is substituted at the 2' position with a C₁ to C₄ alkoxy-Ci to C₄ alkyl group is referred to as a 2'-O-( C₁ to C₄ alkoxy - C₁ to C₄ alkyl) RNA, e.g., 2'-0-methoxyethyl RNA (MOE RNA).

Another example of a modified ribonucleotide moiety is a ribonucleotide that has a methylene bridge between the 2' -oxygen atom and the 4' -carbon atom. An oligoribonucleotide molecule comprising ribonucleotide moieties that has a methylene bridge between the 2 '-oxygen atom and the 4 '-carbon atom is generally referred to as locked nucleic acid (LNA). See, for example, Kurreck et al, Nucleic Acids Res. 30, 1911-1918 (2002).

Another example of a modified ribonucleotide moiety is a ribonucleotide that is substituted at the 2' position with fluoro group. Such 2'-fluororibonucleotide moieties are known in the art. Molecules comprising 2'-fluororibonucleotide moieties are generally referred to herein as 2'-fluororibo nucleic acids (FANA). See Damha et al, J. Am. Chem. Soc. 120, 12976-12977 (1998).

Modified nucleic acid molecules may also comprise, for example, a peptide nucleic acid moiety, a 2'-fluororibonucleotide moiety, a morpholinophosphoroamidate nucleotide moiety, a tricyclo nucleotide moiety and/or a cyclohexene nucleotide moiety.

Modified nucleic acid molecules may comprise at least 1, 2, 3, 4, 5 or more modified moieties at the 5' end. Additionally or alternatively, said molecules may comprise at least 1, 2, 3, 4, 5 or more modified moieties at the 3' end. Said molecules may comprise a nucleotide cap at the 5' end, the 3' end or both, or between the 5' end and 3' end.

### Detection of nucleic acids

The nucleic acids of the invention may be detected by any method known in the art. Methods for the evaluation of miRNAs in cells are well known and include, for example, hybridisation assays using complementary DNA probes (such as in situ hybridisation using labelled riboprobes, Northern blot, microarrays, ribonuclease protection assays and related techniques) and various nucleic acid amplification assays (such as RT-PCR, and other amplification type detection methods, such as, for example, branched DNA, SISBA, TMA and the like).

Particularly suitable methods include quantitative PCR methods such as RT-PCR, in particular real-time PCR; northern blot analysis and use of microRNA microarrays.

### Antisense nucleic acids and other antagonists

The invention also provides antagonists of the HHV-6 microRNAs disclosed herein. Preferably, an HHV-6 miRNA antagonist binds to said miRNA along at least part of its length, more preferably at the 5' end of the miRNA (the seed region). Even short oligonucletides of, for example, 8 nucleotides can inhibit miRNA function (see Obad et al, Nat Genet 2011, 43:371-378, who show that an 8-nucleotide LNA oligonucleotide binding to the 5' end of an miRNA can inhibit its fuction).

Exemplary antagonists include antisense nucleic acids, such as antagomirs. An antagomir or antagomiR is a small synthetic RNA that is complementary to the specific microRNA target other than having either mispairing at the cleavage site of Ago2 or the introduction of a base modification such as 2' methoxy or phosphorothioate groups to inhibit Ago2 cleavage. The Ago2 cleavage site is located between nuceotides 10 and 11 from the 5' end of the antisense molecule.

Antagomirs may have additional modification such as 2' methoxi groups and phosphothioates, to make them more resistant to degradation, as described above. Antagomir RNAs of the invention will thus preferably have one or more of the modifications described above. Antagomirs may be used to constitutively inhibit the activity of specific microRNAs.

The skilled person will, on the basis of the microRNA sequences disclosed herein, be able to design suitable antagomir or other antisense sequences. Suitable modifications are described above.

Antisense oligonucleotide agents, such as antagomirs, have been shown to function effectively *in vivo* and in culture as specific inhibitors of individual microRNAs and pre-microRNAs (see, for example U.S. Application No. 20070213292; Gottwein el al, Nature 450: 1096-1099 (2007); Krützfeldt el al, Nature 438:685-689 (2005), Krützfeldt et al, Nucleic Acids Research 135:2885-2892 (2007)). Suitable antisense nucleic acids include single-stranded, double-stranded, partially double-stranded or hairpin structured chemically modified oligonucleotides (DNA or RNA) that can include, for example, at least about 8 contiguous nucleotides complementary to, or substantially complementary to (that is, sufficiently complementary that a duplex, can be formed), an HHV-6 microRNA as disclosed herein. Preferably, the antisense nucleic acid includes 8 or more contiguous nucleotides complementary to or substantially complementary to a target sequence of a microRNA or pre-microRNA nucleotide sequence. The length of the antisense nucleic acid can contribute to the biochemical function of the antisense nucleic acid with respect to the ability to decrease expression levels of a specific microRNA. An antisense nucleic acid can be, for example, from about 8 to 30 nucleotides in length, preferably about 15 to 28 nucleotides in length, or may be at least 5, 6, 7, 8, 9, 10, 11, 12, 13, 14, 15, 16, 17, 18, 19, 20, 21, 22, 23, 24, 25, 26, or 27 nucleotides in length or more). In some instances, antisense nucleic acid can require at least 19 nucleotides in length for optimal function.

.An antisense nucleic acid of the invention can be further stabilised against nucleolytic degradation such as by the incorporation of a modification, e.g., a nucleotide modification. In one embodiment, the antagomir can include a 2'-modified nucleotide, e.g., a 2'-deoxy, 2'-deoxy-2'-fluoro, 2'-O-methyl, 2'-O-rnethoxyethyl (2'-0-MOE), 2'-O-aminopropyl (2'-0-AP), 2'-O-dimethy!aminoethyl (2 -O-DMAOE), 2'-0-dimethylaminopropyl (2'-0-DMAP), 2'-O-dimethylaminoethyloxyethyl (2'-0-DMAEOE), or 2'-O~N-methylacetamido (2'-O- NMA), or other modification as described above. In a preferred embodiment, the antisense nucleic acid can include at least one T-O-methyl-modified nucleotide. In yet another preferred embodiment, the antisense nucleic acid can include phosphorothioate backbone modifications. Antisense nucleic acid of the invention can be further modified so as to be attached to a ligand that is selected to improve stability, distribution or cellular uptake.

Other antisense oligonucleotides suitable for use in blocking HHV-6 microRNA expression include locked nucleic acids (Orom et al, Gene 372:137-141 (2006); Elmer et al, Nature 452:896-900 (2008)) and morpholinos (Kloosterman et al, PLoS Biol. 5:pe203 (2007)). An LNA (Locked Nucleic Acid) oligonucleotide is an oligonucleotide comprising one or more LNA nucleotides as described above, which improve the stability and hybridisation properties of the oligonucleotide

### Pharmaceutical compositions and delivery

For pharmaceutical use, the nucleic acids and antagonists of the invention are preferably mixed with a carrier, preferably a pharmaceutically acceptable carrier. 'Carrier' includes solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" or "pharmacologically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared.

Solutions of the nucleic acids of the invention as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intraarterial, intramuscular, subcutaneous, intratumoral and intraperitoneal administration. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, Remington: The Science and Practice of Pharmacy, 21st Edition, Lippincot and Williams, 2005). Some variation in dosage will necessarily occur depending on the condition of the subject being treated. The person responsible for administration will, in any event, determine the appropriate dose for the individual subject.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The compositions disclosed herein may be formulated in a neutral or salt form. Pharmaceutically-acceptable salts, include the acid addition salts and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug release capsules and the like.

Targeting of a microRNA molecule or antagonist thereof to a particular cell can be performed by any method known to those skilled in the art. For example, the microRNA molecule or antagonist thereof can be conjugated to an antibody or ligand specifically recognized by receptors on the cell. For example, if the cell is a B lymphocyte, the antibody can be against the cell receptor CD19, CD20, CD21, CD23 or a ligand to these receptors.

Useful delivery systems include, for example, liposomes and charged lipids. Liposomes typically encapsulate oligonucleotide molecules within their aqueous centre. Charged lipids generally form lipid-oligonucleotide molecule complexes as a result of opposing charges.

These liposomes-oligonucleotide molecule complexes or lipid-oligonucleotide molecule complexes are usually internalised in cells by endocytosis. The liposomes or charged lipids generally comprise helper lipids which disrupt the endosomal membrane and release the oligonucleotide molecules.

Other methods for introducing a microRNA molecule or antagonist thereof into a cell include use of delivery vehicles, such as dendrimers, biodegradable polymers, polymers of amino acids, polymers of sugars, and oligonucleotide-binding nanoparticles. In addition, pluoronic gel as a depot reservoir can be used to deliver the anti-microRNA oligonucleotide molecules over a prolonged period. The above methods are described in, for example, Hughes et al., Drug Discovery Today 6, 303-315 (2001); Liang et al. Eur. J. Biochem. 269 5753-5758 (2002); and Becker et al, Antisense Technology in the Central Nervous System (Leslie, R.A., Hunter, A.J. & Robertson, H.A., eds), pp.147- 157, Oxford University Press.

Vectors such as DNA or RNA plasmid or viral vectors may be used. A particularly preferred vector is a lentiviral vector. Vectors of the present invention can include a multiple cloning site (MCS), which is a nucleic acid region that contains multiple restriction enzyme sites, any of which can be used in conjunction with standard recombinant technology to digest the vector, a promoter which generally comprises a sequence that functions to position the start site for RNA synthesis, and a termination signal which ends the production of an RNA transcript by an RNA vector.

Some microRNA molecule antagonists (e.g. those bearing locked nucleic acid modifications) do not require delivery vehicles and can be introduced naked into a cell *in vitro* or even *in vivo.*

### Diagnosis, prognosis and treatment

The nucleic acids and antagonists described herein may be used in the diagnosis, prognosis and treatment of HHV-6 infections, including latent infections, or conditions related thereto. An 'HHV-6 related condition' may include conditions directly caused by HHV-6 infection, conditions associated with HHV-6 infection, conditions which may be exacerbated by HHV-6 infection, and conditions the development or progression of which may be affected by HHV-6. HHV-6 as used herein may refer to HHV-6A, HHV-6B or all strains of HHV-6 variants. Such conditions include roseola, hepatitis, febrile convulsions, encephalitis and certain tumours including progressive multifocal leukoencephalopathy, and multiple sclerosis.

HHV-6 as used herein may refer to HHV-6A, HHV-6B or all strains of both variants of HHV-6.

The term 'diagnosis' is used herein to refer to the identification of a molecular or pathological state, disease or condition, such as the identification of HHV-6 infection or condition related thereto, such as tumour, or to refer to identification of a patient suffering from HHV-6 infection or condition related thereto who may benefit from a particular treatment regimen. Diagnosis may also involve the detection of a specific HHV-6 subtype, for example HHV-6 A or HHV-6B.

The term 'prognosis' is used herein to refer to the prediction of the likelihood of benefit from therapy. The term 'prediction' or 'predicting' refers to the likelihood that a patient will respond either favourably or unfavourably to a particular therapy. In one embodiment, prediction or predicting relates to the extent of those responses. In one embodiment, the prediction or predicting relates to whether and/or the probability that a patient will survive or improve following treatment, for example treatment with a particular therapeutic agent, and for a certain period of time without disease recurrence. The predictive methods of the invention can be used clinically to make treatment decisions by choosing the most appropriate treatment modalities for any particular patient. The predictive methods of the present invention are valuable tools in predicting if a patient is likely to respond favourably to a treatment regimen, such as a given therapeutic regimen, or whether long-term survival of the patient following a therapeutic regimen is likely.

'Treatment' includes both therapeutic treatment and prophylactic or preventative treatment, wherein the object is to prevent or slow down the targeted pathologic condition or disorder. Those in need of treatment include those already with the disorder as well as those prone to have the disorder or those in whom the disorder is to be prevented. The terms 'therapy', 'therapeutic', 'treatment' or 'treating' include reducing, alleviating or inhibiting or eliminating the symptoms or progress of a disease, as well as treatment intended to reduce, alleviate, inhibit or eliminate said symptoms or progress. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, methods and compositions of the invention are used to delay development of a disease or disorder or to slow the progression of a disease or disorder.

Treatment in accordance with the invention includes a method of treating a cancer or other neoplastic disorder which comprises administering to a patient in need of treatment a protein, vector or pharmaceutical composition of the invention. Preferably, the treatment further comprises administering to said patient a chemotherapeutic drug, preferably a drug in prodrug form. The two components may be administered together, for example in the form of a combined pill, or separately. Administration may be sequential or simultaneous. 'Sequential' administration indicates that the components are administered at different times or time points, which may nonetheless be overlapping. Simultaneous administration indicates that the components are administered at the same time.

Preferably, an effective amount, preferably a therapeutically effective amount of the protein or vector of the invention is administered. An 'effective amount' refers to an amount effective, at dosages and for periods of time necessary, to achieve the desired therapeutic or prophylactic result. The effective amount may vary according to the drug or prodrug with which the protein or vector is co-administered.

A 'therapeutically effective amount' of a protein or vector of the invention may vary according to factors such as the disease state, age, sex, and weight of the individual, and the ability of the protein, to elicit a desired therapeutic result. A therapeutically effective amount encompasses an amount in which any toxic or detrimental effects of the protein are outweighed by the therapeutically beneficial effects. A therapeutically effective amount also encompasses an amount sufficient to confer benefit, e.g., clinical benefit.

In the case of pre-cancerous, benign, early or late-stage tumours, the therapeutically effective amount of the composition of the invention may reduce the number of cancer cells; reduce the primary tumour size; inhibit (i.e., slow to some extent and preferably stop) cancer cell infiltration into peripheral organs; inhibit (i.e., slow to some extent and preferably stop) tumour metastasis; inhibit or delay, to some extent, tumour growth or tumour progression; and/or relieve to some extent one or more of the symptoms associated with the disorder. To the extent the drug may prevent growth and/or kill existing cancer cells, it may be cytostatic and/or cytotoxic. For cancer therapy, efficacy *in vivo* can, for example, be measured by assessing the duration of survival, time to disease progression (TTP), the response rates (RR), duration of response, and/or quality of life.

'Neoplastic disease', 'cancer' and 'tumour' refer to or describe the physiological condition in mammals that is typically characterized by unregulated cell growth. Examples of cancer include carcinoma, lymphoma, blastoma, sarcoma, and leukemia or lymphoid malignancies. More particular examples of such cancers include squamous cell cancer, lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer and gastrointestinal stromal cancer, pancreatic cancer, glioblastoma, gliosarcoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma; chronic lymphocytic leukaemia (CLL); acute lymphoblastic leukaemia (ALL); hairy cell leukaemia; chronic myeloblastic leukaemia; head and neck cancer;and associated metastases, pituitary adenoma and progressive multifocal leukoencephalopathy.. In certain embodiments, cancers that are amenable to treatment by the antibodies of the invention include cancers of the head and neck, leukaemia, lymphoma, gliosarcoma, pancreatic cancer, breast cancer and melanoma.

### BRIEF DESCRIPTION OF THE FIGURES

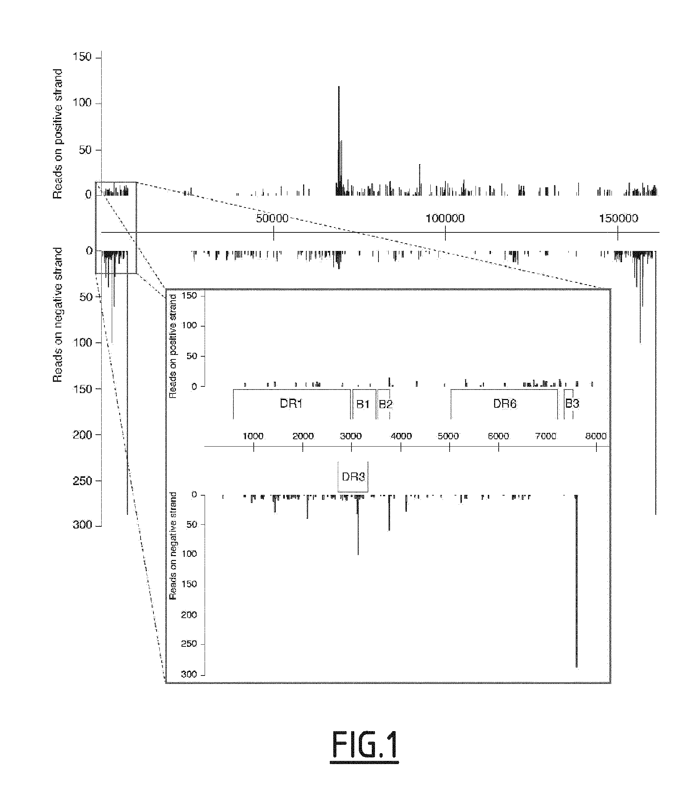

**FIGURE 1****.** Genomic distribution of HHV-6B small RNA sequences. Mapping of small RNA reads (permitting 2 mismatches, and possessing a better match to the pathogen rather to the host) to the Z29 strain of HHV-6B. A. Small RNA cloning from total RNA of SupT1 cells infected with a clinical isolate of HHV-6B (7dpi). The insert zooms into the left direct repeat (DR3), indicating the positions of DR1, DR3 and DR6, along with the HHV-6B specific ORFS: B1, B2, and B3 in respect to the cloned small RNA sequences.

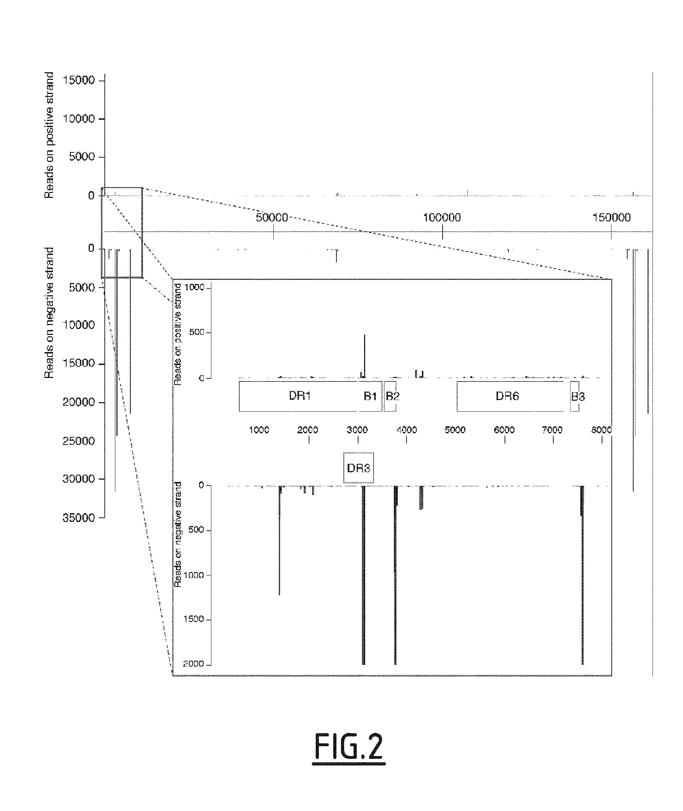

**FIGURE 2****.** Genomic distribution of HHV-6B small RNA sequences. Mapping of small RNA reads (permitting 2 mismatches, and possessing a better match to the pathogen rather to the host) to the Z29 strain of HHV-6B. Representation of small RNA sequences cloned following Ago2 IP using the same viral isolate (4 dpi) across the genome. Three peaks are clearly visible deriving from DR_{L} and DR_{R}, the insert highlights sequences deriving from DR_{L} using a cut-off of 2000 reads.

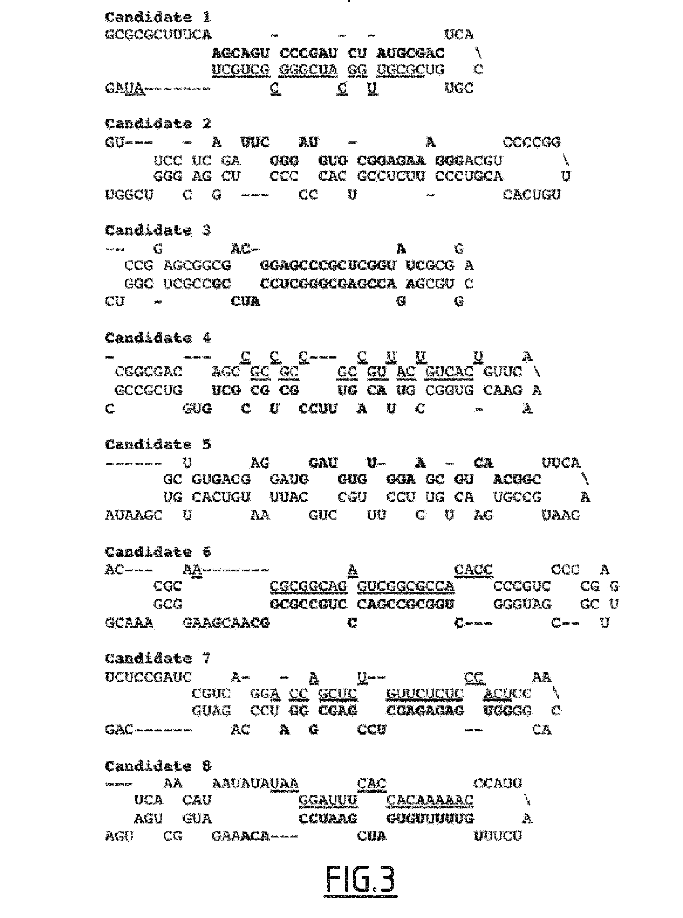

**FIGURE 3**. Predicted structures and validation of HHV-6B microRNAs. Predicted fold-back structures of 8 HHV-6B viral microRNA candidates; in each case the predicted mature microRNA is highlighted in bold, putative star sequences by cloning are underlined.

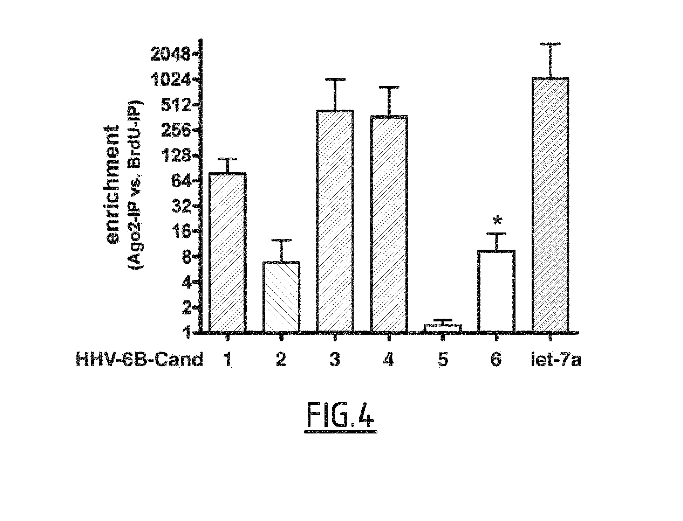

**FIGURE 4****.** Fold enrichment of let-7a (control) and viral sequences within Ago2 IP vs. BrdU controls for certain viral microRNA candidates validates the Northern blot data; a clear enrichment is observed for the microRNA candidates validated as in Ago2 by Northern blot.

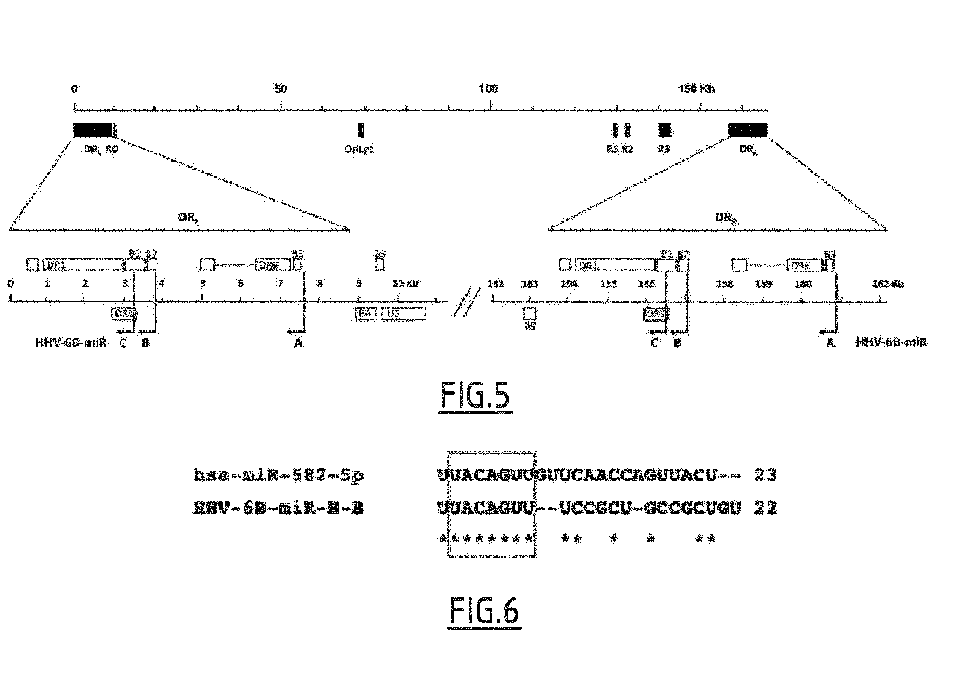

**FIGURE 5****.** Genomic Localisation of HHV-6B microRNAs. The genomic architecture of HHV-6B indicating relative positions of the repetitive regions (DR_{L}, R0, R1, R2 (R2A, R2B), R3 and DR_{R}) and OriLyt is shown, with a zoom-in of each DR region. Direction of transcription is given by placement above (positive), or below (negative) the scale bar. All HHV-6B microRNAs are expressed from 2 positions in the genome, and are transcribed from the negative strand; microRNAs are indicated with arrows and labelled A, B and C, referring to HHV-6B-miR-H-A, H-B, and H-C respectively. DR3 encodes for miR-H-C, and each microRNA is located antisense (either to the coding region, or to the putative 3'-UTR) to an HHV-6B specific ORF that has no homologue with HHV-6A (see text for details).
**FIGURE 6****.** Alignment of HHV-6-miR-H-B with its seed orthologue miR-582-5p; the seed is boxed; homology is indicated by stars (ClustalW). HHV-6B-miR-H-B is a perfect seed-orthologue to a human microRNA, miR-582-5p, that is not expressed in Sup-T-1 cells.

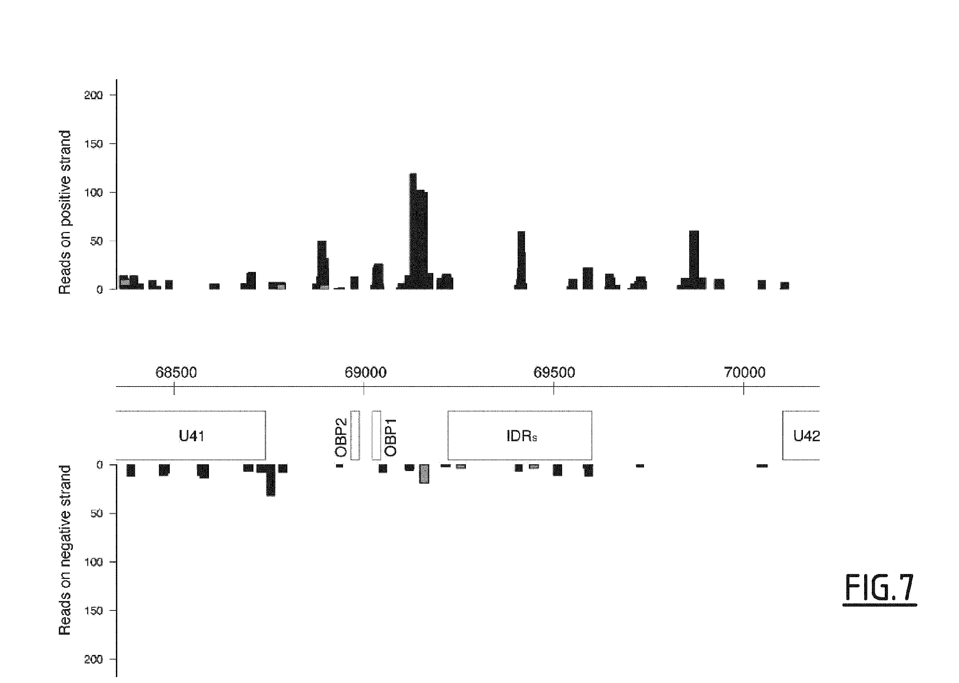

**FIGURE 7**. Small RNAs deriving from the OriLyt are incorporated into Ago2. Representation of small RNA reads mapping to the region encompassing the origin of lytic replication from total RNA infected with HHV-6B (7 dpi). Sites for the origin binding protein (OBP) are given, along with the positions of the indirect repeat regions (IDRs) and neighbouring genes, U41 and U42.

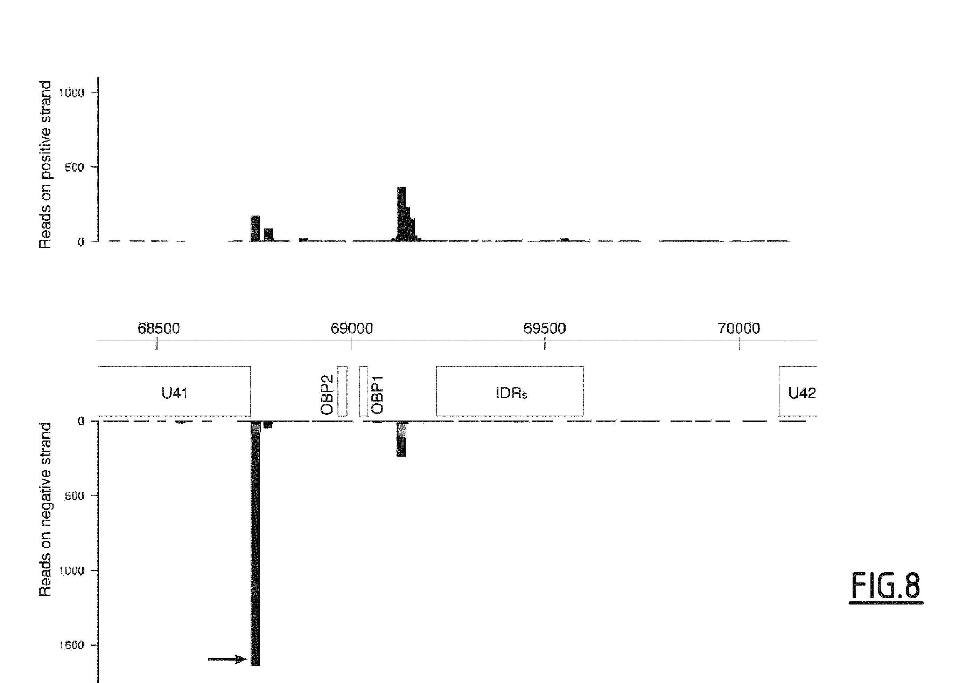

**FIGURE 8****.** Small RNAs deriving from the OriLyt are incorporated into Ago2. Sequences mapping to the OriLyt obtained following sequencing of RNAs within Ago2. A clear enrichment of two sets of antisense sequences is visible following the IP. The leftmost sequence (candidate 8) is enriched - 50 times (compared to Fig. 5A) but has not been detected by Northern blot but likely represents a microRNA produced at low levels. C. Northern blots for two contiguous sequences enriched in Ago2 deriving from a GC rich region in between OBP1 and the IDR. A novel viral transcript encompassing positions 69121-69161 is clearly detectable and increases in abundance throughout the time course of infection. One arm of this transcript generates a range of abundant small RNAs, which are not detectable by Northern blot in Ago2 and may represent a novel class of origin-derived small RNAs.

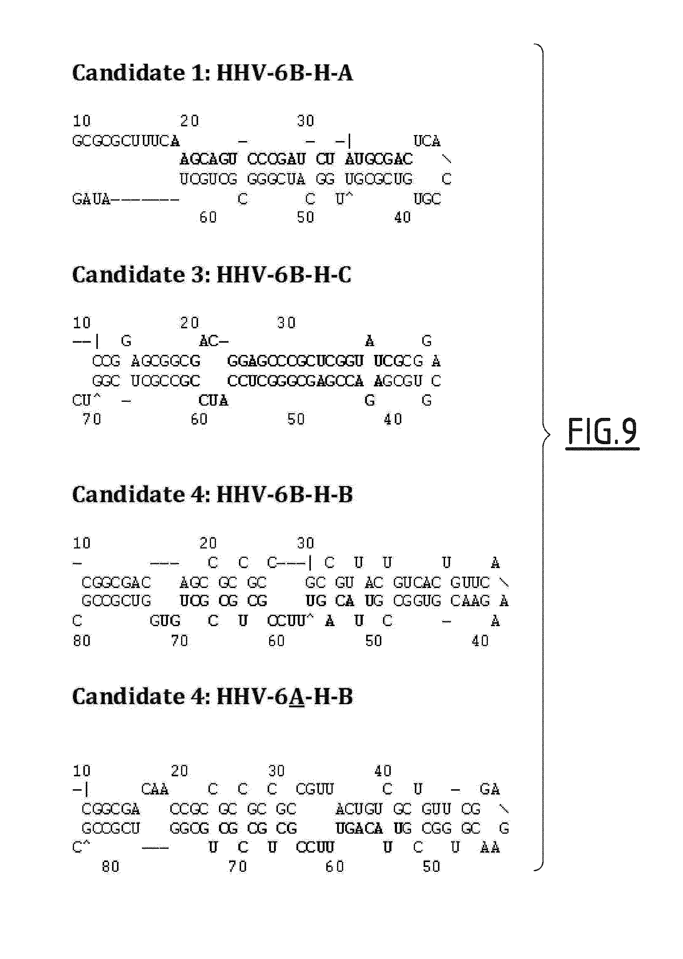

**FIGURE 9**. HHV-6B pre-microRNA structures: intervariant and interstrain variation. Secondary structure predictions for HHV-6B microRNAs based on cloned sequences aligned to the Z29 strain of HHV-6B; microRNAs are highlighted in bold, cloned star sequences are underlined. The predicted precursor for HHV-6B-miR-H-A is fully conserved in both HHV-6B Z29 and HST strains as well as in HHV-6A. HHV-6B-pre-miR-H-B is conserved entirely between HST and Z29 strains, but presents with 91% conservation at the sequence level with HHV-6A. This variability in sequence does not affect the predicted pre-microRNA structure; there is a final base substitution for position 22 of the mature microRNA. Due to the differences at the pre-microRNA level, this microRNA from HHV-6A is represented as a separate structure. HHV-6B-miR-H-C presents with the greatest variability in mature microRNA sequence, and displays interstrain and intervariant differences in the seed region of both the 3p and 5p arms for HHV-6B HST and HHV-6A in relation to the HHV-6B Z29 strain.

### EXPERIMENTS

### Materials and Methods

### Cell culture and virus infection

Sup-T-1 cells (CRL-1942; ATCC) were maintained in RPMI-1640 medium containing 7% fetal bovine serum (FBS). 3x10⁵ cells/mL were infected with HHV-6B (clinical isolate) by co-cultivation of uninfected Sup-T-1 cells with infected cells at a ratio of 1:1000. Samples were harvested at 0, 24, 48, 72 and 96 hours post-infection (hpi), and total RNA was isolated using Trizol (Invitrogen, Carlsbad, CA) according to the manufacturer's recommendations.

### Immunofluorescent staining

For immunofluorescence analysis to determine the rate of HHV-6 infection, 2x10⁵ cells in 150 µL PBS per slide were pelleted on a glass slide (cytospin) at 800 rpm for 10 min. Slides were air dried, fixed and permeabilised with fixative solution for CINAkit 19-002 (Argene, Sherley, USA) and permeabilization solution for CINAkit 19-002 (Argene, Sherley, USA), respectively. Primary antibody, sc-65447 (Santa Cruz) for the fluorescence staining was applied at a concentration of 0.5% for 1 hour, followed by a 1 hour incubation with the secondary donkey anti-mouse Alexa Fluor® 488 antibody (Invitrogen, Darmstadt). Evans blue (1%) was used for counterstaining.

### Immunoprecipitation of human Argonaute 2 (hAgo2) complexes

For the RISC-IPs, 1x10⁸ cells were used for each replicate and washed twice in PBS before lysis in 10 mL of lysis buffer, containing 25 mM Tris HCl pH 7.5, 150 mM KCI, 2 mM EDTA, 0.5% NP-40, 0.5 mM DTT, and complete protease inhibitor (Roche). DTT and protease inhibitors were always prepared freshly and added immediately before use. Lysates were incubated for 30 min at 4°C and cleared by centrifugation at 20,000 g for 30 min at 4°C. Total RNA was prepared from 100 µL of cell lysates using the miRNeasy kit (Qiagen) following the manufacturer's instructions. Ago2-immunoprecipitation was performed as previously described (18). Briefly, 6 pg of purified monoclonal hAgo2-antibody (α-hAgo2; 11A9) or control monoclonal BrdU-antibody (Abcam) was added to 5 mL of RPMI-medium and incubated with 30 µL of Protein-G-Sepharose beads (GE Healthcare) in Pierce centrifuge columns (Thermo Scientific) under constant rotation at 4°C overnight. Columns were drained by gravity flow and washed once with the lysis buffer. Beads were subsequently incubated with 5 mL of cell lysates for 2.5 h under constant rotation at 4°C. After incubation, the beads were washed four times with IP wash buffer (300 mM NaCl, 50 mM Tris HCl pH 7.5, 5 mM MgCl2, 0.1% NP-40, 1 mM NaF) and once with PBS to remove residual detergents. RNA was recovered from the beads by adding 700 µL of Qiazol to the columns. After 5 min, the Qiazol lysates were collected from the columns. This step was repeated once and the Qiazol lysates were combined. RNA was prepared using the miRNeasy kit (Qiagen) according to the manufacturer's instructions. RNA samples were eluted in 30 µL H₂O and stored at -80°C until subjected to further analyses.

### cDAM synthesis and quantitative light-cycler PCR

To monitor the efficiency of the Ago2-IP and check for selective enrichment of HHV-6B microRNA candidates, cDNA was prepared from 2.5 µL Ago2-IP, BrdU-IP and total RNA (1:10) in a single step reaction using the miScript Reverse Transcription kit (Qiagen). Light Cycler qRT-PCR was performed for the cellular microRNA let7a with the following forward primer (let7a for, 5'TGAGGTAGTAGGTTGTATAGTT), or with the respective sequences of the mature HHV-6 microRNA candidates using the miScript SYBR Green PCR kit (Qiagen) following the manufacturer's instructions.

### Small RNA cloning and sequencing

RNA was extracted from non-infected and HHV-6B infected (7 days post infection) Sup-T-1 cells using Tri-reagent (MRC, Inc) as per manufacturer's instructions. Small RNA cloning was conducted from 30 µg of total RNA as previously described (13), except that PCR products were not concateramized, and instead sent directly for large-scale sequencing. Small RNA sequencing was performed at the Institut de Génétique et de Biologie Moléculaire et Cellulaire (IGBMC, Illkirch, France) using an Illumina genome analyser II with a read length of 36 base pairs (bp). Similarly, for deep-sequencing of small RNAs incorporated into Ago2-containing RISC complexes, a small RNA library was performed from hAgo2 IP of Sup-T-1 cells infected with HHV-6B at 4 dpi using a read length of 54 bases.

### Annotation of small RNA sequences

Sequence reads were trimmed of their 3' adaptor sequence and aligned to the following databases: *Homo Sapiens* genome - UCSC assembly version hg19; *Homo sapiens* rRNA, tRNA, scRNA, sn-snoRNA and piRNA - Genbank v. 1.80; Repeats *(Homo sapiens* and common ancestral repeats) - Repbase v. 16.01; microRNAs - miRBase v.16; and for the HHV-6B genome - RefSeq NC_000898.1. using Nexalign permitting up to 2 mismatches.

### Northern Blotting

Total RNA was extracted using Tri-reagent (MRC, Inc) from Sup-T-1 cells infected with HHV-6B (clinical isolate) following 1, 2, 3, and 4 days of infection. Total RNA (10 µg), or 5 µL of BrdU IP or Ago2 IP samples were mixed with an equal volume of formamide loading dye and heated at 95°C for 30 seconds prior to separation on a 17.5% urea-acrylamide gel. RNA was transferred to a HybondNX membrane (Amersham Biosciences) in Milli-Q water and chemically crosslinked at 60°C using 1-Ethyl-3-[3-dimethylaminopropyl]carbodiimide hydrochloride (EDC) (Sigma) for 1hr 30 min followed by extensive washing in Milli-Q water prior to prehybridisation. Membranes were prehybridised for 2 hrs in PerfectHybTM plus (Sigma) at 42°C. For each of the 8 candidate microRNAs, antisense DNA oligos containing 3 locked nucleic acid (LNA) bases (Eurogentec), or standard antisense DNA oligos (for HHV-6B-miR-H-C-5p and sequences antisense to positions 69121-69141 and 69141-69161) were 5' end labelled using T4 polynucleotide kinase (Fermentas) with 25 µCi of [γ-32P]dATP. The labelled probe was hybridised to the blot overnight at 42°C. The blot was then washed at 55°C twice for 10 min with low stringency buffer (5 x SSC/0.1 % SDS), followed by a single wash in a higher-stringency buffer (1 x SSC/0.1 % SDS) for 5 mins. Northern blots were exposed overnight to phosphorimager plates (Fuji) and scanned using a FLA-5000 series phosphorimager (Fuji).

### Results

### Identification of HHV-6B-derived small RNAs

To identify viral microRNAs encoded by HHV-6B, the inventors performed small RNA cloning of Sup-T-1 cells infected with a laboratory strain of HHV-6B followed by deep-sequencing. Sup-T-1 is a CD4⁺ T-lymphocyte cell line that is permissive to HHV-6, HHV-7, as well as to HIV-1 infection (19, 20). Total RNA isolated from Sup-T-1 cells at 7 days post infection (dpi) with HHV-6B was used for the generation of small RNA libraries. HHV-6 infection was confirmed by immunofluorescence staining against the HHV-6 p41 early antigen. Total RNA from HHV-6B infected cells was size fractionated on a polyacrylamide gel, and RNA species between 19 and 24 nucleotides were cloned as previously described (Dunn et al. (2005) Cell Microbiol, 7, 1684-1695; Boyd et al (1993). J Virol, 67, 3649-3652). In total, 24,380,617 reads were obtained of which 93.9% could be mapped to either the human or HHV-6B genome, and were categorized into RNA classes based on sequence annotation (Table. 1). An unusually high number of reads were annotated as rRNA (71.58%). Most likely, this is due to non-synchronized, prolonged infection, i.e. infected cells were added to uninfected cells at 1:1000, and thus reduced RNA quality. Similar findings were previously noted for small RNA libraries generated from HCMV infected cells. Allowing two mismatches for the alignment to the viral genome, only a limited number of reads (12,350, 0.05%) could be assigned as viral sequences. Of these, 9138 sequences mapped preferentially to the HHV-6B Z29 genome.

To enhance sensitivity and boost the degree of confidence in our analysis, the inventors performed small RNA cloning and deep-sequencing analysis from RNAs obtained after Ago2 immunoprecipitation (Ago2 IP). In total, 30,225,650 reads were obtained, of which 97.93% were mappable. It was observed that ~ 95% of all reads were annotated as microRNAs; a few more may have been missed due to their misannotation as snoRNAs (22). The prevalence of microRNAs within Ago2 confirmed that the majority of sequences deriving from the virus (207,394 reads, 0.69%) represent genuine viral microRNAs (Table. 1). Such a limited number of viral sequences is interesting when compared to results for other herpesviruses, particularly of the betaherpesvirus family where viral microRNAs constituted 10-60% of the cellular microRNA pool late in infection.

### Distribution of HHV-6B sequences across the genome

To simplify the analysis and reduce noise, only sequences with zero mismatches to HHV-6B (Z29 strain), between 19 and 24 nucleotides (with no corresponding human genomic hit) were used to search for putative microRNA candidates. Sequences were mapped to the HHV-6B Z29 reference genome (NC_000898.1) to look for hotspots across the genome. The HHV-6B genome consists of a unique long region (U) of 145 kb, flanked by two identical repetitive regions of 8 to 9 kb that contain direct repeats (DR_{L} and DR_{R}). The distribution of HHV-6B small RNA sequences across the genome was quite striking. Mapping of HHV-6B sequences revealed that the majority of viral sequences derive from either the repetitive direct repeat regions, or from the region between ORFs U41 and U42 that contains the origin of lytic replication (OriLyt) (24). Entire genomic representations of cloned sequences from both total RNA (Fig. 2) and from RNA obtained from Ago2 IP (Fig. 3) are given in reference to the Z29 strain. Comparing whole genome reads between total RNA and Ago2 IP RNA, a distinct enrichment in certain sequences deriving from the direct-repeat regions was observed (FIG. 3). Reads mapped from sRNA cloning of total RNA clearly define a central peak, which correlates with the origin of lytic replication (OriLyt) (Fig. 2). Sequences deriving from in or around the OriLyt of both rat and mouse cytomegalovirus (RCMV and MCMV respectively) have previously been reported to encode for microRNAs or postulated that they may act as RNA primers for the replication of the viral genome.

### Identification of HHV- 6 microRNAs

Those sequences that were cloned multiple times and had characteristic 5' homogeneity were assessed manually using Mfold (27) for their ability to fold into typical stem-loop precursor structures. Eight sequences (named candidates 1-8) deriving from eight putative classical pre-microRNA stem-loop structures that matched perfectly to the HHV-6B Z29 genome were initially selected for validation (FIG. 3). To validate these candidates, Northern blot analysis of RNA taken from a time-course of HHV-6B infection from 1 to 4 days post infection (dpi) was performed, using non-infected Sup-T-1 cells as control. In addition, to verify if these were *bona fide* microRNAs RNA samples from Ago2 IPs (and BrdU IP samples as controls) from both non-infected and infected cells (4 dpi) were loaded. Three of these sequences (candidates 1, 3 and 4) were nicely validated as novel HHV-6B microRNAs by their presence in Ago2 by Northern blotting; both the 3p and 5p forms of candidate 3 were detected in Ago2, and were both detectable by Northern blot.

Although sequences representing candidates 2, 6, 7 and 8 were readily cloned (237, 289, 1214, and 1639 times respectively) from the Ago2 IP, the inventors were unable to formally validate them as microRNAs. While candidates 2, 7 and 8 remained undetectable in both total RNA and Ago2-IP by Northern blot, candidate 6 strongly accumulated during the time-course of infection as two distinct RNA species but remained undetectable in the Ago2-IP; of note, a small non-specific signal was detected in the control.

The inventors further verified these results by using quantitative real-time PCR to identify enrichment of the small viral RNAs in Ago2 vs. BrdU controls; consistent with the Northern blot data, qPCR revealed significant enrichment of candidates 1, 3 and 4 in the Ago2-IPs (Fig. 4). Candidates 2 and 6 were significantly enriched in the Ago2-lPs compared to BrdU-IP controls, although the signal for candidate 6 was weak and non-specific, reflecting the Northern blot data (Figs 3 and 4). Candidate 5 was cloned from total RNA, and was not present in the Ago2 IP RNA-Seq data, but was selected as a candidate as it derived from a non-repetitive genomic region and folded into a hairpin structure. No enrichment in Ago2 was observed for candidate 5 by qPCR, confirming the sequencing data; no signal was observable by Northern blot (Fig. 4 and data not shown).

These mRNAs have been preliminarily named as HHV-6B-miR-H-A (candidate 1), HHV-6B-miR-H-B (candidate 4), and HHV-6B-miR-H-C-3p and -C-5p (candidates 3-3p and 3-5p). These microRNAs are referred to herein by their letter classifiers. The sequences of the mature microRNAs and their star sequences are given in Table 2, along with the number of times they were cloned from both total RNA and Ago2 IP libraries. The sequences of the predicted pre-microRNAs as shown in Fig. 3) are given in Table 3 in respect to the Z29 strain of HHV-6B.

### Characteristics of HHV- 6 microRNAs

A schematic representation of HHV-6B microRNAs in respect to their genomic localisation in the direct repeats is given in Figure 5. Of note, miR-HHV-6B-H-C is encoded antisense to the predicted B1 ORF, whilst HHV-6B-H-B is encoded within DR3 and lies antisense to the predicted ORF, B2. B1 and B2 are unique to HHV-6B among all herpesviruses, and were recently identified as 2 of the 8 immediate-early (IE) genes expressed by HHV-6B via transcriptional analyses. The function of B1 and B2 are unknown, but is thought that due to their classification as IE genes, and their specificity to HHV-6B that they may play important roles in HHV-6B specific infection. The identification of viral microRNAs that are transcribed antisense to viral genes is now a well described feature of known viral microRNAs, proven regulation of antisense targets have been defined for: EBV, HCMV, HSV-1 and HSV-2, and for polyomaviruses.

Interestingly, such viral microRNAs do not always regulate their antisense targets by cleavage as is the case for SV40-miR-1 and its target, the large-T antigen (35), but may inhibit their targets by the classical translational inhibition model, as is the case for HCMV-miR-UL112-1 and its target UL114. It thus appears that HHV-6B uses HHV-6B-miR-H-B and HHV-6B-miR-H-C-3p to down-regulate B2 and B1 respectively. Of note, HHV-6B-miR-H-B bridges the 3' coding region and the presumed 3'-UTR of B2 with the 3p and 5p arms of its precursor. To our knowledge, such a feature has not been previously described for any viral or cellular microRNA. As for HHV-6B-miR-H-B, HHV-6B-miR-H-C also lies antisense to an immediate early transcript (B2), but is encoded within DR3. DR3 is a gene of unknown function which is expressed as early as 6h following infection, and is blocked by cycloheximide (CHX) treatment (28). As DR3 has not been classified in terms of its kinetics, we presume that DR3 and HHV-6B-miR-H-C share similar kinetics, although it is possible that both these microRNAs are transcribed by an upstream promoter as they share the same expression pattern for both the mature and precursor microRNA forms. Finally, HHV-6B-miR-H-A, which was the most abundant microRNA identified from sequencing of total RNA (7 dpi) (Table 2), was only detectable following 3 dpi (2 days later than the other HHV-6B microRNAs). Therefore, it may be assumed that all three of these microRNAs are not produced from one large transcript, but that at least HHV-6B-miR-H-A is transcribed from a separate promoter. As for the microRNAs located antisense to B1 and B2, HHV-6B-miR-H-A is encoded antisense to the potential 3'-UTR of B3, an HHV-6B specific gene of unknown function that has been classified as either an IE, or early gene. In general, all HHV-6B microRNAs have the potential to regulate HHV-6B specific genes, which is an unusual feature of these microRNAs. Currently, no antibodies are available against B1 to B3. In addition to these particular features of HHV-6B microRNAs, HHV-6B-miR-H-B is a seed orthologue (bases 2-8) of the cellular microRNA miR-582-5p (Fig. 6). Northern blots to detect the poorly conserved miR-582-5p from non-infected and infected cells failed to detect the expression of this cellular microRNA in Sup-T-1 cells (data not shown). This is in accordance with the sequencing data, in which miR-582-5p was absent. This microRNA is up-regulated in certain pituitary adenomas, and has provisionally been linked with targeting SMAD3 to down-regulate TGF-β; further validation is however required. It is possible that that, analogous to KSHV-miR-K12-11 (a microRNA which is highly expressed in primary effusion lymphoma (PEL), though its seed orthologue (miR-155) is absent), HHV-6B-miR-H-B may share a broad range of targets with miR-582-5p and function in cells that do not normally express this cellular microRNA to modulate the host-pathogen environment.

### Conservation of HHV-6 microRNAs

As a laboratory strain was used for the infection, the inventors performed an additional analysis by re-annotating the small RNA libraries against the HST strain of HHV-6B to look for potential interstrain variations in the validated microRNA sequences. HHV-6B-miR-H-A and HHV-6B-miR-H-B share identical pre-microRNA sequences in both strains, whilst there are notable interstrain variations of HHV-6B-miR-C (Fig 9). HHV-6B-miR-C-5p (not an original candidate due to having a mismatch in respect to the Z29 genome) matches perfectly to HST, but bears one mismatch (C to U) at position 7 (position 6 of the seed) to Z29. Inversely, miR-HHV-6B-miR-C-3p matches Z29 perfectly, but is altered (G to A) at position 2 (seed position 1); hairpin structures are unaffected by these differences. The relevance of these interstrain variations is unknown, and would require careful sequencing analysis of all known strains of HHV-6B. However, variations in the microRNA sequence, particularly in the seed region may contribute to functional differences in respect to their cellular targets. In effect, with the predicted targeting of their antisense transcripts, interstrain variations would not affect the regulation of the relevant viral antisense transcripts. In terms of intervariant variation, HHV-6B and HHV-6A share over 90% sequence homology, although demonstrate greater diversity within genes encoding for IE-1 and within the terminal direct repeats. All HHV-6B microRNAs are conserved within HHV-6A at both the structural and sequence level, although they display minor variations in their sequences (Fig. 9). In addition, the inventors cloned several distinct isoforms of each microRNA that demonstrate 5' and 3' heterogeneity (Table 4). In contrast, the HHV-6B-miR-H-B sequence is perfectly conserved but presents with significant differences within the predicted pre-microRNA. These differences may have functional consequences in respect to the different biological properties of these two viruses.

### Other small RNAs identified in HHV-6

A general analysis of small viral RNAs in Ago2 indicates that the three microRNA hairpins verified as HHV-6B microRNAs comprise ~ 80% of all viral sequences. Considering that ~ 95% of all cellular RNAs were classifiable as microRNAs, by convention, there are likely additional rarer viral microRNAs, which have been not confirmed by this study. Of note, candidates 7 and 8 form fold-back hairpins (Fig. 3) with characteristic 5' homogeneity, and were enriched in libraries generated from Ago2 compared to total RNA (Figs 2, 3, 7 and 8). We include these as potential microRNAs awaiting validation by Northern blot; sequences and genomic positions can be found in Table 4. A breakdown of the distribution of sequence reads across the genome identifies ~ 85% deriving from the direct repeats (DR), ~ 7.5% from OriLyt, and ~ 7.5% from elsewhere. As mentioned, microRNAs have been verified for both MCMV and RCMV from in or around their OriLyt regions. As such, Northern blots were performed for the three most abundant sequences deriving from the origin: candidate 8 on the negative strand, (position c68744-68765), and two contiguous sequences that do not form a typical hairpin that are located on the positive strand (69121-69141 and 69141-69161). Interestingly, probes against the contiguous sequences identified a novel viral transcript of ~ 62 nt spanning this region (see Table 6). Of note, sequencing data from both libraries indicate multiple RNA species deriving from this locus (sequences in Ago2 ranging from 17 to 32 nucleotides); and this is clearly visible as a broad set of vertical bars in the graphical representation of OriLyt sequences and by a smear on the Northern blot (Figs 7 and 8 and data not shown). Of note, abundant RNA species of 18 and 19 nt are identified only from arm of the precursor, the signal indicates that these are not in Ago2 at comparable levels to total RNA samples (as seen for viral microRNAs). As such, their presence in Ago2 cloning data may represent a non-specific binding of Ago2 due to their abundance within the cell. However, such a postulation is unlikely in light of the quality of the sequencing data. It is possible that these small RNAs derive from a novel viral RNA that is specifically degraded to generate small RNA species that may represent RNA primers to assist in viral replication. Alternatively, they could be a new class of small RNAs that are not wholly incorporated into Ago2, but may be processed by another member of the Argonaute family.

In addition to those microRNAs identified, sequencing data indicate that several thousand unique, low abundant sequences are associated with Ago2. Due to their low frequency, it is likely that these represent RNA breakdown products that non-specifically associate with Ago2, and are likely due to the associated issues with non-synchronized infection. We also identify for each of the three microRNAs, that we clone sequences located antisense to the microRNAs, albeit in extremely low levels (Fig. 2). This has been previously reported for some KSHV microRNA, and this is also the case for candidates 7 and 8, giving credence to their candidacy as true viral microRNAs.

**Table 1. Distribution of small RNA reads from both libraries**

| *The origin of cloned small RNAs in the small RNA libraries is indicated in percentage from the total clones numbers. Abbreviations: rRNA, ribosomal RNA; tRNA, transfer RNA; sn-snoRNA, small nuclear-small nucleolar RNA; scRNA, small cytoplasmic RNA.* | | |
|---|---|---|
| | **HHV-6B** | |
| **RNA** | **Total** | **HHV-6B** |
| **Classification** | **RNA** | **Ago2-IP** |
| rRNA | 71.58 | 0.29 |
| tRNA | 1.12 | 0.25 |
| sn-snoRNA | 1.00 | 0.45 |
| scRNA | 0.14 | 0.09 |
| microRNAs | 11.97 | 94.96 |
| Repeats | 0.93 | 0.13 |
| Host genome | 6.82 | 1.03 |
| HHV-6B (Z29) | 0.05 | 0.69 |
| genome | | |
| Unknown | 6.38 | 2.11 |
| Total % | 100.00 | 100.00 |

**Table 2. HHV-6B microRNA sequences, genomic positions and distribution in the two libraries**

| *The predominantly cloned sequence is indicated. 5p, 5' arm of the hairpin precursor; 3p, 3' arm of the hairpin precursor; *, non-functional star sequence. Positions are given relative to the published genomic sequence of the Z29 strain of HHV-6B.* | | | | | | | | |
|---|---|---|---|---|---|---|---|---|
| **miRNA HHV-6B** | **Cloned sequence** | **Strand** | **Size** | **No. of reads** | **No. of reads in Ago2** | **Genomic location DR_{L}** | **Genomic position DR_{R}** | **SEQ ID No** |
| miR-H-A | AAGCAGUC CCGAUCUA UGCGAC | - | 22 | 287 | 25449 | [7590-7611] | [160911-160932] | 1 |
| miR-H-A* | CGCGUUGG CAUCGGGC GCUGCUAU | | 24 | 1 | 946 | [7557-7580] | [160878-160901] | 2 |
| miR-H-B | UUACAGUU UCCGCUGC CGCUGU | - | 22 | 110 | 55321 | [3754-3775] | [157076-157096] | 3 |
| miR-H-B* | CGCCGCCG CCGUUACU GUCACU | | 22 | 2 | 612 | [3794-3815] | [157115-157136] | 4 |
| miR-H-C-5p | GACGGAGU CCGCUCGG UAUCG | - | 21 | 100 | 21460 | [3128-3148] (1) | [156449-156469] (1) | 5 |
| miR-H-C-3p | AGACCGAG CGGGCUCC AUCCG | - | 21 | 32 | 61900 | [3097-3117] | [156418-156438] | 6 |

**Table 3. HHV-6B precursor microRNAs sequences and genomic positions.**

| *The indicated sequences correspond to the sequences used to make the predicted foldback structures shown in Fig. 3A. Nucleotides highlighted in bold indicate the mature microRNA sequences. Positions are given relative to the published genomic sequence of the Z29 strain of HHV-6B.* | | | | | |
|---|---|---|---|---|---|
| **Pre- miRNA HHV-6B** | **Sequence** | **Strand** | **Genomic location DR_{L}** | **Genomic location DR_{R}** | **SEQ ID No** |
| pre-miR- H-A | GCGCGCUUUCAAGCA GUCCCGAUCUAUGCG ACUCACCGUGUCGCG UUGGCAUCGGGCGCU GCUAUAG | - | [7555-7621] | [160876-160942] | 7 |
| pre-miR- H-B | CGGCGACAGCCGCCG CCGCCGUUACUGUCA CUGUUCAAAGAACGUG GCCGUUACAGUUUCC GCUGCCGCUGUGGUC GCCGC | - | [3745-3825] | [157066-157146] | 8 |
| pre-miR- H-C | CCGGAGCGGCGACGG AGCCCGCUCGGUAUC GCGGACGUGCGAGAC CGAGCGGGCUCCAUC CGCCGCUCGGUC | - | [3087-3157] | [156408-156479] | 9 |

**Table 4. Major isoforms of HHV-6B microRNAs**

| *Sequences of major isoforms of HHV 6B microRNAs are given along with their genomic position in respect to the left direct repeat region (DR_{L}). Only those sequences cloned over 1000 times and found within Ago2 are represented in the table. HHV-6B microRNA can present with 5' and 3' heterogeneity, as wells as present as shorter isoforms lacking a part of the 5' region of the microRNA. Sequences are given in reference to the Z29 strain of HHV-6B; mm, mismatches are given in parentheses.* | | | | | | | |
|---|---|---|---|---|---|---|---|
| **HHV-** | **Reads** | **Length** | **Sequence** | **Strand** | **Start** | **End** | **SEQ ID** |
| **6B-miR** | | | | | | **(mm)** | **No** |
| | | | | | | | |
| miR-H-A | 21403 | 22 | 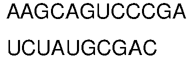 | - | 7590 | 7611(0) | 1 |
| miR-H-A | 1281 | 21 | AGCAGUCCCGAU CUAUGCGAC | - | 7590 | 7610(0) | 10 |
| | | | | | | | |
| miR-H-B | 24242 | 22 | UUACAGUUUCCG CUGCCGCUGU | - | 3754 | 3775(0) | 3 |
| miR-H-B | 14090 | 21 | UUACAGUUUCCG CUGCCGCUG | - | 3755 | 3775(0) | 11 |
| miR-H-B | 5029 | 22 | UUACAGUUUCCG CUGCCGCUGA | - | 3754 | 3775(1) | 12 |
| miR-H-B | 4513 | 16 | UUUCCGCUGCC GCUGU | - | 3754 | 3769(0) | 13 |
| | | | | | | | |
| miR-H- | | | 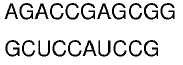 | | | | 6 |
| C-3p | 31540 | 21 | | - | 3097 | 3117(0) | |
| miR-H- | | | AGACCGAGCGG GCUCCAUCC | | | | 14 |
| C-3p | 11489 | 20 | | - | 3098 | 3117(0) | |
| miR-H-C-3p | 2862 | 17 | CGAGCGGGCUC CAUCCG | - | 3097 | 3113(0) | 15 |
| miR-H-C-3p | 2096 | 18 | CCGAGCGGGCU CCAUCCG | - | 3097 | 3114(0) | 16 |
| miR-H-C-3p | 1655 | 22 | AGACCGAGCGG GCUCCAUCCGA | - | 3096 | 3117(1) | 17 |
| miR-H-C-3p | 1090 | 22 | AGACCGAGCGG GCUCCAUCCGC | - | 3096 | 3117(0) | 18 |
| miR-H-C-3p | 1004 | 20 | GACCGAGCGGG CUCCAUCCG | - | 3097 | 3116(0) | 19 |
| | | | | | | | |
| miR-H-C-5p | 10293 | 21 | GACGGAGUCCG CUCGGUAUCG | - | 3128 | 3148(1) | 5 |
| miR-H-C-5p | 2237 | 20 | GACGGAGUCCG CUCGGUAUC | - | 3129 | 3148(1) | 20 |
| miR-H-C-5p | 1977 | 22 | ACGGAGUCCGCU CGGUAUCGCG | - | 3126 | 3147(1) | 21 |
| miR-H-C-5p | 1109 | 22 | GACGGAGUCCG CUCGGUAUCGC | - | 3127 | 3148(1) | 22 |
| | | | | | | | |

**Table 5. Sequences of additional candidates**

| Sequences representing the major form of each candidate are presented, original candidates 2, 5, and 6 were not validated as microRNAs, although candidates 2 and 6 may represent rare microRNAs, hence their inclusion. Candidate 7 (DR) and candidate 8 (OriLyt) represent the most abundant sequences which form hairpin stem loops that have not been verified as microRNAs, although are predicted as microRNAs due to their abundance in Ago2. Viral sequences are given in reference to the Z29 strain of HHV-6B, sequences numbers are from Ago2 IP data; *candidate sequence not identified in Ago2 IP. | | | | | | | |
|---|---|---|---|---|---|---|---|
| **Candidate** | **Reads** | **Size** | **Sequence** | **Strand** | **Start** | **End (mm)** | **SEQ ID No** |
| 2 | 98 | 23 | UUCGGGAUGU GCGGAGAAAG GGA | - | 2083 | 2105(0) | 23 |
| 5 * | 3 | 24 | UGGAUGUGUG GAAGCGUCAAC GGC | - | 27024 | 27047(0) | 24 |
| 6 | 80 | 23 | GCUGGCGCCG ACCCUGCCGC GGC | + | 4318 | 4340(0) | 25 |
| 7 | 1214 | 22 | GGUGAGAGAG CUCCGAGCGG GA | - | 1392 | 1413(0) | 26 |
| 8 | 1639 | 22 | 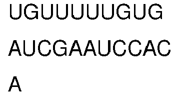 | - | 68744 | 68765(0) | 27 |

**Table 6: Novel viral transcript spanning the OriLyt region, located on the positive strand (69121-69182).**

| **Sequence** | **Size** | **Strand** | **Start** | **End** | **SEQ ID No** |
|---|---|---|---|---|---|
| AGAGGGACGCTGGGGT ACGACTTGAGACGTTTG ACTGAAAATGATCCTTC GTGTACTATTTT | 62 | + | 69121 | 69182 | 28 |

The inventors have shown using a deep-sequencing approach that HHV-6B encodes for at least 3 microRNAs expressed during lytic infection that are all conserved in HHV-6A, and potentially encodes for additional rarer microRNA species as evidenced by the identification of numerous small RNA species being sequenced from Ago2 IPs. The use of sRNA cloning from Ago2 IPs simplifies the analysis by removing the majority of RNA break down products, providing a clean dataset to aid in identifying novel viral microRNAs. Three microRNAs accumulate during infection and are encoded antisense to IE HHV-6B specific ORFS (B1, B2 and B3), following the paradigm that viral microRNAs regulate IE genes to modulate their own transcription. As for KSHV-miR-K12-11 and miR-155, HHV-6B-miR-H-B encodes a seed orthologue of a cellular microRNA (miR-582-5p) that is not expressed in the main target cell of the virus, in this case the CD4⁺ T- cell line Sup-T-1. These data provide good starting points for identifying the cellular and viral targets of these microRNAs. As with HCMV, HHV-6 is a β-herpesvirus and its microRNAs were identified in cells undergoing active lytic infection.
Unlike all other human herpesviruses, HHV-6A has been categorically proven to integrate into host telomeres as a method of latency, rather than residing as an episome, importantly the virus can be reactivated from this state, and be passed on through generations. It is thus possible that these microRNAs are expressed in latently infected cells as well as in chromosomally integrated HHV-6 (CIHHV-6).

## Claims

1. An isolated nucleic acid molecule comprising the sequence of a HHV-6 microRNA or the complement thereof, or an antagonist thereof, wherein said HHV-6 microRNA has a sequence shown in Tables 2 to 6 (SEQ ID Nos 1-28).

2. An antagonist according to claim 1, which antagonist is an antisense nucleic acid comprising a sequence which is the complement of a sequence shown in Tables 2 to 6 (SEQ ID Nos 1-28).

3. A method of diagnosis of an HHV-6 infection or an HHV-6-related condition, said method comprising detection of an nucleic acid according to claim 1 in a biological sample from a patient.

4. A method according to claim 3, wherein said HHV-6 related condition is multiple sclerosis.

5. A method of prognosis of patients suffering from a non-HHV-6 viral infection, in particular a HHV-7, HCMV, or HIV-1 infection, which method comprises detection of nucleic acid according to claim 1 in a biological sample from said patient, wherein detection of said nucleic acid is associated with a poorer prognosis of said infection.

6. A nucleic acid or antagonist according to claim 1 or claim 2 for use in a method of treating an HHV-6 infection or condition related to an HHV-6 infection.

7. A nucleic acid for use according to claim 6, wherein said treatment is aimed at prevention of graft rejection in a transplant recipient.

8. A vector comprising a nucleic acid according to claim 1 or claim 2.

9. A method of overexpression of a nucleic acid according to claim 1 or claim 2 comprising introducing a vector according to claim 8 under conditions suitable for overexpression of said nucleic acid.

10. An *in vitro* method of inhibiting microRNP activity, said method comprising contacting said microRNP or a cell comprising said microRNP with an HHV-6 antagonist according to claim 1 or claim 2.

11. An *in vitro* method of down-regulating expression of an HHV-6 or cellular protein in a cell, said method comprising introducing a nucleic acid of claim 1 into said cell, wherein said nucleic acid is a HHV-6B-miR-H-A microRNA (SEQ ID No 1 or 10), a HHV-6B-miR-H-B microRNA (SEQ ID No 3, 11, 12 or 13), a HHV-6B-miR-H-C-3p microRNA (SEQ ID No 6, 14, 15, 16, 17, 18, or 19), or a HHV-6B-miR-H-C-5p microRNA (SEQ ID No 5, 20, 21 or 22).

12. A method according to claim 11, wherein said protein is HHV-6 B1, B2 or B3 protein.

13. A method of identifying a candidate HHV-6 microRNA antagonist, said method comprising contacting a test compound with a nucleic acid according to claim 1 and determining the presence or absence of a complex comprising said test compound and said nucleic acid, wherein the presence of said complex is indicative of the suitability of said test compound as an HHV-6 antagonist.


SEQUENCE LISTING

<110>  CNRS

<120>  MicroRNAs from Human Herpesvirus 6

<130>  BET11P2235

<160>  28

<170>  PatentIn version 3.5

<210>  1
<211>  22
<212>  RNA
<213>  HHV6

<400>  1
aagcaguccc gaucuaugcg ac                                                22

<210>  2
<211>  24
<212>  RNA
<213>  HHV6

<400>  2
cgcguuggca ucgggcgcug cuau                                              24

<210>  3
<211>  22
<212>  RNA
<213>  HHV6

<400>  3
uuacaguuuc cgcugccgcu gu                                                22

<210>  4
<211>  22
<212>  RNA
<213>  HHV6

<400>  4
cgccgccgcc guuacuguca cu                                                22

<210>  5
<211>  21
<212>  RNA
<213>  HHV6

<400>  5
gacggagucc gcucgguauc g                                                 21

<210>  6
<211>  21
<212>  RNA
<213>  HHV6

<400>  6
agaccgagcg ggcuccaucc g                                                 21

<210> 7
<211> 67
<212> RNA
<213> HHV6

<400> 7
gcgcgcuuuc aagcaguccc gaucuaugcg acucaccgug ucgcguuggc aucgggcgcu 60

gcuauag 67


<210> 8
<211> 81
<212> RNA
<213> HHV6

<400> 8
cggcgacagc cgccgccgcc guuacuguca cuguucaaag aacguggccg uuacaguuuc 60

cgcugccgcu guggucgccg c 81


<210> 9
<211> 72
<212> RNA
<213> HHV6

<400> 9
ccggagcggc gacggagccc gcucgguauc gcggacgugc gagaccgagc gggcuccauc 60

cgccgcucgg uc 72


<210> 10
<211> 21
<212> RNA
<213> HHV6

<400> 10
agcagucccg aucuaugcga c 21


<210> 11
<211> 21
<212> RNA
<213> HHV6

<400> 11
uuacaguuuc cgcugccgcu g 21


<210> 12
<211> 22
<212> RNA
<213> HHV6

<400> 12
uuacaguuuc cgcugccgcu ga 22


<210> 13
<211> 16
<212> RNA
<213> HHV6

<400>  13
uuuccgcugc cgcugu                                                       16


<210>  14
<211>  20
<212>  RNA
<213>  HHV6

<400>  14
agaccgagcg ggcuccaucc                                                   20


<210>  15
<211>  17
<212>  RNA
<213>  HHV6

<400>  15
cgagcgggcu ccauccg                                                      17


<210>  16
<211>  18
<212>  RNA
<213>  HHV6

<400>  16
ccgagcgggc uccauccg                                                     18


<210>  17
<211>  22
<212>  RNA
<213>  HHV6

<400>  17
agaccgagcg ggcuccaucc ga                                                22


<210>  18
<211>  22
<212>  RNA
<213>  HHV6

<400>  18
agaccgagcg ggcuccaucc gc                                                22


<210>  19
<211>  20
<212>  RNA
<213>  HHV6

<400>  19
gaccgagcgg gcuccauccg                                                   20


<210>  20
<211>  20
<212>  RNA
<213>  HHV6

<400>  20

gacggagucc gcucgguauc                                                    20

<210>  21
<211>  22
<212>  RNA
<213>  HHV6

<400>  21
acggaguccg cucgguaucg cg                                                 22

<210>  22
<211>  22
<212>  RNA
<213>  HHV6

<400>  22
gacggagucc gcucgguauc gc                                                 22

<210>  23
<211>  23
<212>  RNA
<213>  HHV6

<400>  23
uucgggaugu gcggagaaag gga                                                23

<210>  24
<211>  24
<212>  RNA
<213>  HHV6

<400>  24
uggaugugug gaagcgucaa cggc                                               24

<210>  25
<211>  23
<212>  RNA
<213>  HHV6

<400>  25
gcuggcgccg acccugccgc ggc                                                23

<210>  26
<211>  22
<212>  RNA
<213>  HHV6

<400>  26
ggugagagag cuccgagcgg ga                                                 22

<210>  27
<211>  22
<212>  RNA
<213>  HHV6

<400>  27
uguuuuugug aucgaaucca ca                                                 22

<210>  28
<211>  62
<212>  DNA
<213>  HHV6

<400>  28
agagggacgc tggggtacga cttgagacgt ttgactgaaa atgatccttc gtgtactatt        60

tt                                                                        62